# EUROPEAN PATENT APPLICATION

(11) **EP 4 268 718 A1**
(43) Date of publication of application: **01.11.2023**
(21) Application number: 22170717.7
(22) Date of filing: 29.04.2022
(51) Int. Cl.: A61B 5/145, A61B 5/16

(54) **VIRTUAL REALITY SYSTEM**

(71) Applicant: BIC Violex Single Member S.A., 145 69 Anoixi (GR)
(72) Inventor: Antonakis, Ion - Ioannis, 14569 Anoixi (GR)
(74) Representative: Peterreins Schley

(57) **Abstract**

There is provided a virtual reality headset system. The virtual reality headset system comprises a display device, a headband coupled to the display device, and a sensor configured to measure an indication of at least one biomarker in the sweat of a user associated with a stress level of the user when the user is wearing the virtual reality headset system.

## Description

### TECHNICAL FIELD

The embodiments described herein relate to a virtual reality headset system and an associated computer-implemented method for monitoring at least one biomarker in the sweat of a user during using of the virtual reality headset system.

### BACKGROUND

With the rise of Ed Tech (Technologies in Digital Education), digital fatigue has emerged in the educational domain, with significant physical and psychological impacts on both teachers and students. A device or a process that would reduce and/or mitigate these impacts would be welcomed by consumers.

Digital fatigue is directly correlated with stress; recent studies have shown that there is a 10% increase in stress levels at younger ages in the period between 2013 to 2019. On the other hand, multiple studies have shown that stress can also aid in the learning process as the hormones released during a stressful event can enhance memory formation, thus leading to robust memories. However, this effect is active up to a certain threshold, which when exceeded can undermine the learning process.

Virtual Reality (VR) has been in the forefront of technological developments with multiple companies developing complete product solutions. Consumer awareness of the technology is increasing, and this is reflected by the fact that VR headset sales are expected to grow by more than double by 2024. This increase in sales has also triggered research in Human Machine Interfaces (HMI) for the VR platform. Many products that track users' finger and wrist motions are available and can be used for VR applications. Most recently, engineers and researchers from ETH Zurich have developed a handwriting digitization device for VR applications (including drawing), that is essentially mimicking a physical writing instrument. The user is holding a physical pen, that is then brought inside the virtual world. Such a device can improve the drawing experience in a VR environment, given that today is limited due to the use of entire VR controllers as an input system.

A substantial amount of research has also been focused on the effects of VR on its users. Recent studies have indicated that VR can both distract users from pain, while also reducing their stress levels significantly. Moreover, PwC has reported that VR in education can contribute to the user's learning abilities since VR learners were found to absorb information four times faster than their counterparts in a real classroom.

Recently, research has increasingly focused on the development of biosensors to measure stress, which is not necessarily based solely on measuring heartbeat or an increase in body temperature. For this purpose, hormones that are present in sweat may be used.

A suitable indicator for stress is cortisol. Cortisol is a steroid hormone that historically has been difficult to measure without a blood sample. A research team from Caltech has managed to develop a nonintrusive method for measuring cortisol levels in human sweat. This ultrathin graphene sensor can accurately determine the stress levels of a user - given that cortisol is directly correlated to stress in humans. A very thin graphene structure is doped with cortisol antibodies, that trap the cortisol found in the user's sweat, which then can be detected electrochemically.

Further, a recent study has shown users can significantly reduce their cortisol (stress) levels, after following an art-making session, irrespective of their prior experience and type of artmaking.

The preceding findings lead to the situation that VR headsets can be further developed in terms of monitoring the stress of the user and thus be made suitable for a number of applications.

### SUMMARY

According to a first aspect, there is provided a virtual reality headset system. The virtual reality headset system comprises a display device, a headband coupled to the display device, and a sensor configured to measure an indication of at least one biomarker in the sweat of a user associated with a stress level of the user when the user is wearing the virtual reality headset system.

According to a second aspect, there is provided a computer-implemented method for monitoring at least one biomarker in the sweat of a user during using a virtual reality headset system. The computer-implemented method comprises providing a first virtual reality content to the user through a virtual reality headset system, measuring the concentration level of the at least one biomarker in the sweat of a user through a sensor coupled to the virtual reality headset, interrupting the providing of the first virtual reality content to the user, when the concentration level of the at least one biomarker exceeds a predetermined first threshold concentration level, and returning to providing the first virtual reality content to the user, when the concentration level reaches a level below a predetermined second threshold concentration level.

An effect of the technique of the present specification is to provide a virtual reality headset system that is configured to measure an indication of at least one biomarker in the sweat of a user associated with a stress level and thus may open a wide range of applications, for example, to reduce stress level of students in a digital education environment. This may make the latest findings on good learning situations applicable in practice. The proposed headset allows digital education environments that are more customized to a student's individual needs or cognitive abilities than conventional forms of teaching. A possible minimizing of potential physical and psychological impacts on both teachers and students due to elevated stress levels may allow a more efficient learning and may enhance memory formation.

The proposed virtual reality headset system may improve the learning abilities of students by allowing them to maintain an optimal level of stress, which can be achieved not least by interrupting learning sessions when measured biomarkers indicate excessive stress.

The suggested interruptions of a learning session can build further skills of the student. For example, one effect may be to improve the student's artmaking skills by using interruptions for digital art teaching content. Progressive monitoring of a stress-indicating biomarker even during the interruption may provide the ability to return to an educational environment when the stress level of the user has normalized.

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different figures indicate like or similar features.
- **Figure 1**: schematically illustrates an example of a virtual reality headset system with a sensor configured to measure an indication of at least one biomarker in the sweat of a user associated with a stress level of the user.
- **Figure 2**: schematically illustrates an example of a virtual reality input interface.
- **Figure 3**: schematically illustrates an example of a virtual reality input interface with an exemplary communication interface.
- **Figure 4**: schematically illustrates an example of a computer-implemented method for monitoring at least one biomarker in the sweat of a user.
- **Figure 5**: schematically illustrates an example of an exemplary calibrating step before providing a first virtual reality content to the user.

### DETAILED DESCRIPTION

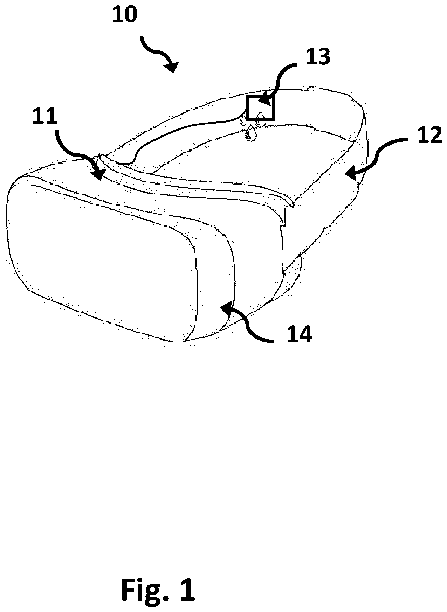

**FIG. 1** schematically illustrates an example of a virtual reality headset system 10. According to the first aspect, the virtual reality headset system 10 comprises a display device 11, a headband 12 coupled to the display device 11, and a sensor 13 configured to measure an indication of at least one biomarker in the sweat of a user associated with the stress level of the user, when the user is wearing the virtual reality headset system 10. A "virtual reality" may be a simulated experience via the display device 11 that can be similar to or completely different from the real world. Applications of virtual reality may include entertainment (particularly video games), education (such as medical or military training), and business (such as virtual meetings). Other distinct types of VR-style technology may include augmented reality and mixed reality, sometimes referred to as extended reality or XR. The display device 11 may be configured to display (virtual reality) content to the user wearing the virtual reality headset system 10.

In an embodiment, the at least one biomarker is a stress hormone. In an example, the at least one biomarker is cortisol. Cortisol is a steroid hormone that is produced by the adrenal glands and is released by the human body in the presence of a stressful event. Thereby, cortisol is present in sweat in a similar concentration as in blood. The stress level experienced by a person is directly correlated with the cortisol level.

In an embodiment, the sensor 13 may be configured to measure a concentration level of the at least one biomarker and to output a signal corresponding to the concentration level. In an example, a higher voltage or current may be generated at the output of the sensor 13 when the concentration level of the biomarker in the sweat of the user increases, or for example, a lower voltage or current may be generated at the output when the concentration level of the biomarker in sweat decreases, or vice versa. In an example, the sensor 13 comprises a graphene layer, for example, a single layer carbon structure, with cortisol antibodies configured to bound the cortisol in the sweat of the user allowing to output a signal corresponding to the cortisol concentration level. In an example, a higher voltage or current may be generated at the output of the sensor 13 when the concentration level of cortisol in the sweat of the user becomes higher or for example, a lower voltage or current may be generated at the output when the concentration level of cortisol in sweat decreases, or vice versa.

In an example, the virtual reality headset system 10 may comprise a power supply (not shown) configured to provide electrical power. The sensor 13 and/or the display device 11 may be configured to receive electrical power from the power supply. The power supply may comprise an energy storage device, such as a lithium-ion battery, or any other suitable form of energy storage device. Further, the power supply may comprise a charging system configured to charge the energy storage device, or any kind of a power supply circuit configured to provide electrical power directly to the components, such as sensor 13 and/or display device 11, of the virtual reality headset system 10. The charging system and/or the power supply circuit may comprise an AC-DC converter, for example.

In an example, the sensor 13 may be attached to a location at the body of the user where sensor 13 may contact the skin of the user, such as head, arms, hands, legs, feet, or torso. For example, the sensor 13 may be part of a self-adhesive pad that may be attached to a suitable location, preferably, in the locations where the human body secretes more sweat than in other locations.

In an embodiment, the sensor 13 may be attached to the headband 12 or to the display device 11 at a location where the sensor 13 may contact the skin of the user, such as the forehead, neck, back of the head, temples, chin area, nose, area around the eyes, end or the upper head area. The possible design of the virtual reality headset system 10 and wearing it on the head of the user may cause the user to start sweating during use. Particularly at the points where the virtual reality headset system 10 rests or rests on the user's head, it may be suitable to place the sensor 13.

In an embodiment, the display device 11 may be one of, but not limited to, a liquid crystal display (LCD), an organic light emitting diode display (OLED), or a light emitting diode display (LED). The display device 11 may be configured to use a (variable) refresh rate of for example ≥ 60 Hz, ≥ 72 Hz, ≥ 90 Hz, ≥ 120 Hz, ≥ 240 Hz, ≥ 360 Hz. The display device 11 may be part of a mobile device that may be inserted into the virtual reality headset 10 by the user before using the headset.

In an embodiment, the virtual reality headset 10 may comprise a computer system 14 configured to operate the virtual reality headset and to receive a signal corresponding to the indication of the at least one biomarker. In an example, the sensor 13 may be electrically connected to an interface of the computer system to provide the signal corresponding to the concentration level of the at least one biomarker to the computer system 14. In an example, the sensor 13 may be electrically connected to a sensor communication interface, such as a near field communication chip, being placed close to the sensor 13 or mechanically coupled to the sensor 13 and configured to transmit the signal corresponding to the concentration level to the computer system 14. The computer system 14 may comprise at least one of a central processing unit (CPU), a random access memory (RAM), or a read only memory (ROM). In an example, the computer system 14 may comprise a storage medium configured to store software, firmware and/or data required to operate the virtual reality headset system 10. Encompassed in the definition of a storage medium, but not limited to, is long-term storage such as flash storage or a hard disc drive capable of storing the firmware, and software required for the computer system 14 to store, read, and process digital data. The computer system 14 may comprise software such as the operating system (OS) that is configured to manage the hardware and software resources and provides common services for application specific algorithms. The computer system 14 may be configured to process the signal corresponding to the concentration level of the at least one biomarker. The computer system 14 may further be configured to provide driving data to the display device 11, for example through a display interface encompassing the electronics for converting the output of the central processing unit to a video signal suitable for visual representation by the display device 11 such that the user may experience the virtual environment or the virtual content when using the virtual reality headset system 10.

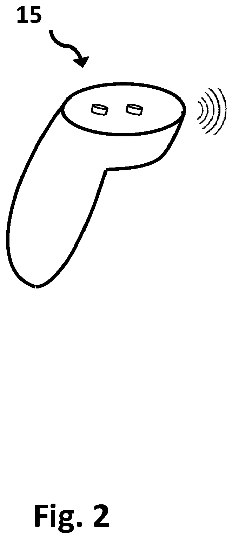

**Fig. 2** schematically illustrates an example of a virtual reality input interface 15.

In an embodiment, the virtual reality headset system 10 may comprise a virtual reality input interface 15 configured to track movements of the user and to output a signal corresponding to the movements. The virtual reality input interface 15 may be electrically coupled to another interface of the computer system 14 to transmit signals to other components of the computer system 14, such as the CPU, by wiring, or may be configured to communicate by wireless connection, for example using Bluetooth or Wireless Fidelity (Wi-Fi) as a communication protocol. The computer system 14 may be configured to process the output signal corresponding to the movements from the virtual reality input interface 15 and may be configured to coordinate the hardware of the headset 10 such that the user requests (input) are recognized and executed appropriately. The computer system 14 may comprise hardware and software to allow displaying content to the user and to interact with the virtual environment and/or virtual reality applications provided through the display device 11 to the user via the virtual reality input interface 15. The interface 15 may be configured to track movements of the user such as hand movements, arm movements, entire body movements, and/or eye movements, and may be configured to translate the physical movements into data that may be utilized by the computer system 14 as an input and may be further processed. The virtual reality input interface 15 may comprise motion trackers, such as Microsoft Kinect, Joysticks, HTC Vive Controllers, Oculus Rift Controllers, Generic VR controllers.

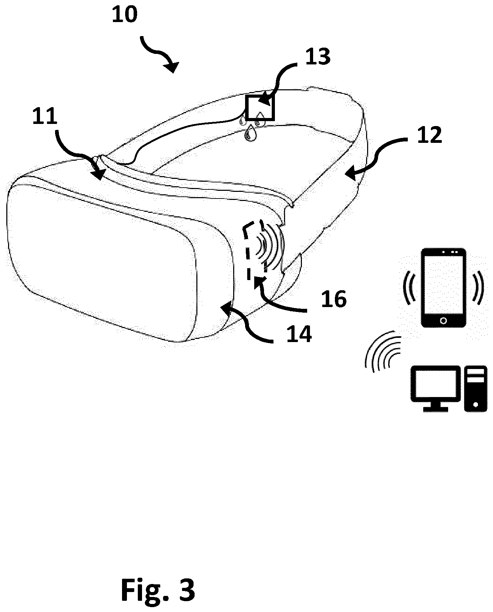

**Fig. 3** schematically illustrates an example of a virtual reality input interface 15 with an exemplary communication interface 16.

In an embodiment, the computer system 14 may comprise a communication interface 16 configured to allow the computer system 14 to communicate with a computer and/or a mobile device. The communication interface 16 may be configured to establish the wireless connection between the virtual reality headset system 10 and other devices (such as a computer and/or a mobile device). The communication protocol operated by the communication interface 16 may be, for example, a Bluetooth or Wireless Fidelity (Wi-Fi) interface, as but two examples. The communication interface 16 may be configured to communicate with the virtual reality input interface 15 to receive the signals corresponding to the movements of the user and/or may be configured to communicate with the sensor 13 using wireless communication through a sensor communication interface.

In an example, sensor 13 may be further configured to measure the heart rate of the user associated with the stress level of the user, when the user is wearing the virtual reality headset system 10 and the sensor 13 is contacting the skin. The sensor 13 may be configured to provide a signal corresponding to the heart rate to the computer system 14. The computer system 14 may be configured to process the data corresponding to the heart rate of the user in an equal manner as the data corresponding to the concentration level of the at least biomarker. In an example, the sensor 13 configured to measure the heart rate of the user may be one of an optical heart rate sensor utilizing photoplethysmography or an electrical heart rate sensor utilizing electrocardiography.

In an example, the sensor 13 may be further configured to measure the body temperature of the user associated with the stress level of the user, when the user is wearing the virtual reality headset system 10. The sensor 13 may be configured to measure the body temperature directly on the skin of the user and/or may be configured to measure the temperature of the sweat of the user indicating the body temperature. The sensor 13 may be configured to provide a signal corresponding to the body temperature to the computer system 14. The computer system 14 may be configured to process the data corresponding to the body temperature of the user in an equal manner as the data corresponding to the concentration level of the at least biomarker. In an example, the at least biomarker may be a body temperature.

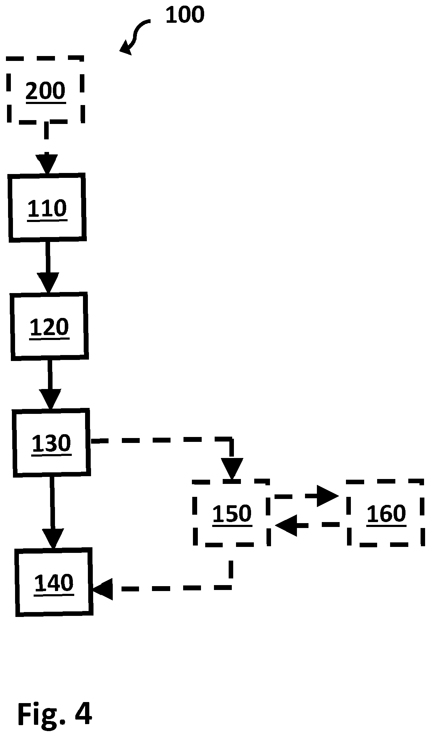

**Fig. 4** schematically illustrates an example of a computer-implemented method 100 for monitoring at least one biomarker in the sweat of a user.

According to the second aspect, there is provided a computer-implemented method 100 for monitoring at least one biomarker in the sweat of the user during using a virtual reality headset system 10. The computer-implemented method comprises providing 110 a first virtual reality content to the user through a virtual reality headset system 10, measuring 120 the concentration level of the at least one biomarker in the sweat of the user through a sensor 13 coupled to the virtual reality headset system 10, interrupting 130 the providing 110 of the first virtual reality content to the user, when the concentration level of the at least one biomarker exceeds a predetermined first threshold concentration level, and returning 140 to providing the first virtual reality content to the user when the concentration level reaches a level below a predetermined second threshold concentration level. The first virtual reality content may be a virtual educational content, preferably in an asynchronous virtual educational environment, where each student can work at their own pace, or in addition to an educational environment in the physical world. The virtual educational environment may be a digital classroom or other kinds of auditoriums or workshop environments. The environment may also be an examination environment. In an example, the concentration level of the at least one biomarker exceeds a predetermined first threshold concentration level because of a situation and/or an event experienced by the user triggering a stress level of the user being higher than a stress level before the situation and/or event. In an educational environment, as the course progresses, the user might have some questions or may have not completely comprehended the thought material. This can potentially lead to a rising stress level of the user, for example, as the user might think that the user is "left behind". The increased stress causes the body of the user to release specific biomarkers indicating stress, such as cortisol, in the bloodstream of the user, which is then reflected to the sweat of the user. When the concentration level of the at least biomarker exceeds a predetermined first threshold concentration level the user may be notified by the system to pause the learning and the providing 110 of the first virtual reality content is interrupted. Once a period of time has passed and the concentration level has moved below a predetermined second threshold concentration level, the system returns 140 to provide the first virtual reality content.

In an embodiment, the computer-implemented method 100 may comprise providing 150 a second virtual reality content to the user configured to reduce the concentration level of the at least one biomarker in the sweat of the user, when providing the first virtual reality content to the user is interrupted. While interrupting the providing 110 of the first virtual reality content the system may provide a second virtual reality content to the user which is suitable for reducing the concentration level of the at least one biomarker associated with the stress level of the user. Such a virtual reality content may comprise instructions on how to draw predefined shapes/sketches/drawings or any other kind of artmaking task. The artmaking task may comprise drawing on a digital canvas, a virtual reality sculpting session, a virtual reality pottery session, or a painting tutorial. The artmaking task may be tailored to the experience of each user and preferred artmaking type. The second virtual content is based on the age of the user and/or skills of the user. The second virtual reality content may comprise relaxing virtual environments such as environments known from holidays or virtual landscapes that appeal to the user. Any kind of virtual reality content may be used that is suitable for lowering the concentration level of the at least biomarker associated with the stress level of the user.

In an embodiment, the computer-implemented method may comprise adjusting 160 the second virtual reality content, when the concentration level may be not reduced at a predetermined rate. The adjusting 160 may comprise simplifying a task to be solved by the user, improving the comprehensibility of the second virtual reality content, and/or temporal extending of the virtual reality content. During the second virtual reality content configured to reduce the concentration level of the at least one biomarker the concentration level may be constantly monitored by the system. If the concentration level of the at least one biomarker maintains over the second threshold concentration level for a predetermined time range and/or does not reduce at a predetermined rate, the artmaking task, for example, may be changed and/or simplified to ensure that the concentration level of the at least one biomarker, such as cortisol, associated with a stress level of the user is being reduced to allow to return 140 to providing 110 the first virtual reality content.

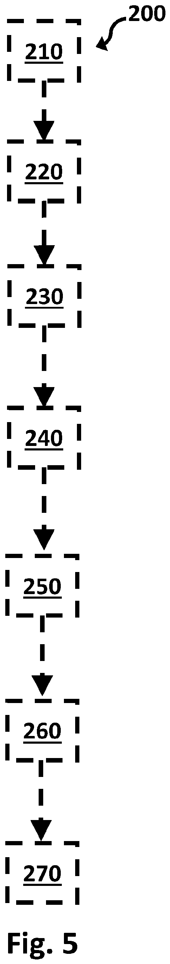

**Fig. 5** schematically illustrates an example of an exemplary calibrating step before providing 110 the first virtual reality content to the user.

In an embodiment, the computer-implemented method 100 may comprise calibrating 200 before providing the first virtual reality content to the user. The calibrating 200 may comprise providing 210 a plurality of virtual situations to the user, measuring 220 the concentration level of at least one biomarker in the sweat of the user corresponding to each virtual situation of the plurality of virtual situations by the sensor 13, receiving 230, via an input interface 15, from a user an estimation of the stress level felt by the user corresponding to each virtual situation of the plurality of virtual situations, matching 240 the estimation of a stress level felt by the user with the concentration level measured by the sensor 13 corresponding to each virtual situation of the plurality of virtual situations, determining 250 a third threshold concentration level corresponding to a stress level above which the user has experienced a stress level in a smaller number of virtual situations that is greater than a stress level experienced by the user in a greater number of other virtual situations of the plurality of virtual situations and/or determining 260 a fourth threshold concentration level corresponding to a stress level below which the user has experienced a stress level in a smaller number of virtual situations that is smaller than a stress level experienced by the user in a greater number of other virtual situations of the plurality of virtual situations, storing 270 the matching, the third threshold concentration level and/or the fourth threshold concentration level in the storage medium. Given that each user might have different cortisol responses to a similar stressful event, the calibrating 200 may be required to relate the stress level experienced by the user to the measured concentration level of the at least one biomarker. For example, a series of stressful tasks can be given to the user while constantly monitoring the cortisol level. At the end of each task, the user may be asked to grade the task in terms of the stress the user felt. This grading may be used by the system to create an appropriate relationship between the concentration level of the at least one biomarker and the subjectively perceived stress level. In an example, the calibrating 200 is enabled in predefined time intervals or based on a quality criterion derivable from the results of measuring the concentration level of the at least one biomarker. In an example, while using the virtual reality headset system 10, the frequency of interrupting 130 in a given time period may be recorded. For example, a quality criterion can be derived from the fact interrupting 110 occurs more frequently in a certain time period than in previous time periods, which indicates that calibrating 200 may be necessary. For example, calibrating 200 may be performed every hour, several times an hour or day, or randomly. For example, calibrating may be performed whenever a new first virtual reality content may be provided. In an example, the first virtual reality content may comprise a plurality of various educational content. For example, calibrating 200 may be performed between a first virtual educational content and a second virtual educational content.

In an embodiment, the first threshold concentration level may equal the third threshold concentration level, or may be below the third threshold concentration level, or may be above the third threshold concentration level. Calibrating 200 may be used to determine the threshold above which a user may perceive a task or virtual situation to be more stressful than another virtual situation, which may be determined using a concentration level of the biomarker that may be greater than a concentration level corresponding to a greater number of other virtual situations of the plurality of virtual situations. For example, the intake of the user of the first virtual content, such as educational content, may be hindered by a perceived stress level. The third threshold concentration level, which may be determined during calibrating 200, may result in the first threshold concentration level above which the providing 110 of the first virtual content is interrupted, but it can also only represent a rough order of magnitude in which, for example, the first threshold concentration level may be arranged.

In an embodiment, the second threshold concentration level may equal the fourth threshold concentration level, or may be below the fourth threshold concentration level, or may be above the fourth threshold concentration level. Calibrating 200 may be used to determine the threshold concentration level below which a user may perceive a task or virtual situation to be less stressful than another virtual situation, which may be determined using a concentration level of the biomarker that may be smaller than a concentration level corresponding to a greater number of other virtual situations of the plurality of virtual situations. For example, while interrupting 130 the providing 110 of the first virtual reality content, the concentration level of the at least one biomarker falls below a second threshold concentration level. The fourth threshold concentration level, which may be determined during calibrating 200, may result in the second threshold concentration level below returning 140 to providing 110 the first virtual reality content is executed, but it can also only represent a rough order of magnitude in which, for example, the second threshold concentration level may be arranged. In an example, the first threshold concentration level and the second threshold concentration level are approximately the same. For example, when the first threshold concentration level is exceeded, the providing 110 may be interrupted and when the concentration level falls again below the first threshold concentration level returning 140 to providing 110 may be executed.

In an example, the range between a third threshold concentration level and the fourth threshold concentration level forms a range being suitable for a user for learning purposes. For example, each user may have a range between two concentration levels of the at least one biomarker being expressive of the stress level felt by the user that favors a higher receptivity than when the stress level is higher or lower, this may be the case for example in the context of an educational content.

References throughout the preceding specification to "one embodiment", "an embodiment", "one example" or "an example", "one aspect" or "an aspect" means that a particular feature, structure, or characteristic described in connection with the embodiment or example is included in at least one embodiment of the present disclosure. Thus, appearances of the phrases "in one embodiment", "in an embodiment", "one example" or "an example", "one aspect" or "an aspect" in various places throughout this specification are not necessarily all referring to the same embodiment or example.

Furthermore, the particular features, structures, or characteristics can be combined in any suitable combinations and / or sub-combinations in one or more embodiments or examples.

### Embodiments:

1. A virtual reality headset system 10, comprising
   a display device 11;
   a headband 12 coupled to the display device 11; and
   a sensor 13 configured to measure an indication of at least one biomarker in the sweat of a user associated with a stress level of the user, when the user is wearing the virtual reality headset system 10.
2. The virtual reality headset system 10 according to embodiment 1, wherein the at least one biomarker is a stress hormone.
3. The virtual reality headset system 10 according to embodiment 1 or 2, wherein the at least one biomarker is cortisol.
4. The virtual reality headset system 10 according to any one of the preceding embodiments, wherein the sensor 13 is configured to measure a concentration level of the at least one biomarker and to output a signal corresponding to the concentration level.
5. The virtual reality headset system 10 according to any one of embodiments 1 to 4, wherein the sensor 13 comprises a graphene layer with cortisol antibodies configured to bound cortisol in the sweat of the user allowing to output a signal corresponding to the cortisol concentration level.
6. The virtual reality headset system 10 according to any one of the preceding embodiments, further comprising a power supply configured to provide electrical power.
7. The virtual reality headset system 10 according to any one of the preceding embodiments, wherein the power supply comprises an energy storage device configured to store energy.
8. The virtual reality headset system 10 according to embodiment 7, wherein the power supply comprises a charging system configured to charge the energy storage device.
9. The virtual reality headset system 10 according to any one of the preceding embodiments, wherein the sensor 13 is attached to a location at the body of the user where the sensor 13 contacts the skin of the user such as head, arms, hands, legs, feet, torso.
10. The virtual reality headset system 10 according to any one of the preceding embodiments, wherein the sensor 13 is attached to the headband 12 or the display device 11 at a location where the sensor 13 contacts the skin of the user such as the forehead, neck, back of the head, temples, chin area, nose, area around the eyes, and/or the upper head area.
11. The virtual reality headset system 10 according to any one of the preceding embodiments, wherein the display device 11 comprises one of a liquid crystal display, organic light-emitting diode display, or a light-emitting diode display.
12. The virtual reality headset system 10 according to any one of the preceding embodiments, further comprising a computer system 14 configured to operate the virtual reality headset system 10 and to receive a signal corresponding to the indication of the at least one biomarker.
13. The virtual reality headset system 10 according to embodiment 12, wherein the computer system 14 comprises at least one of a central processing unit (CPU), a random access memory (RAM), or a read only memory (ROM).
14. The virtual reality headset system 10 according to embodiment 12 or 13, wherein the sensor is electrically coupled to a sensor communication interface being configured to transmit the signal corresponding to the concentration level to the computer system 14.
15. The virtual reality headset system 10 according to any one of the embodiments 12 to 14, wherein the computer system 14 comprises a storage medium configured to store software, firmware, and/or data required to operate the virtual reality headset.
16. The virtual reality headset system 10 according to embodiment 15, wherein the storage medium comprises at least one of flash storage or a magnetic storage.
17. The virtual reality headset system 10 according any one of the embodiments 12 to 16, wherein the computer system 14 comprises a virtual reality input interface 15 configured to track movements of the user and to output a signal corresponding to the movements.
18. The virtual reality headset system 10 according to embodiment 17, wherein the computer system 14 is configured to process software, wherein the software allows displaying content to the user and/or allows the user to interact with a virtual reality environment and/or virtual reality applications through the virtual reality input interface 15.
19. The virtual reality headset system 10 according to embodiment 17 or 18, wherein movements of the user comprise arm movements, leg movements, head movements, entire body movements and/or eye movements.
20. The virtual reality headset system 10 according to embodiments 17, 18, or 19, wherein the virtual reality input interface 15 comprises motion trackers, joysticks, HTC Vive controllers, Oculus Rift controllers, Generic VR controllers.
21. The virtual reality headset system 10 according to any one of the embodiments 17 to 20, wherein the computer system 14 is configured to process the output signal corresponding to the movements of the user.
22. The virtual reality headset system 10 according to any one of the embodiments 12 to 21, wherein the computer system 14 comprises a communication interface 16 configured to allow the computer system 14 to communicate with a computer and/or a mobile device.
23. The virtual reality headset system 10 according to embodiment 22, wherein the communication interface 16 uses a Bluetooth or WireLess Fidelity (WiFi) communication protocol.
24. The virtual reality headset system 10 according to any one of the preceding embodiments, wherein the sensor 13 is configured to measure the heart rate of a user associated with the stress level of the user, when the user is wearing the virtual reality headset system 10.
25. The virtual reality headset system 10 according to embodiment 24, wherein the sensor 13 configured to measure the heart rate of a user is one of an optical heart rate sensor utilizing photoplethysmography or an electrical heart rate sensor utilizing electrocardiography.
26. A computer-implemented method 100 for monitoring at least one biomarker in the sweat of a user during using of a virtual reality headset system 10, comprising
   providing 110 a first virtual reality content to the user through a virtual reality headset system 10;
   measuring 120 the concentration level of the at least one biomarker in the sweat of the user through a sensor 13 coupled to the virtual reality headset system 10;
   interrupting 130 the providing 110 of the first virtual reality content to the user, when the concentration level of the at least one biomarker exceeds a predetermined first threshold concentration level; and
   returning 140 to providing 110 the first virtual reality content to the user, when the concentration level reaches a level below a predetermined second threshold concentration level.
27. The computer-implemented method according to embodiment 26, wherein the concentration level of the at least one biomarker exceeds a predetermined first threshold concentration level because of a situation and/or an event experienced by the user triggering a stress level of the user being higher than a stress level before the situation and/or event.
28. The computer-implemented method 100 according to embodiment 26 or 27, further comprising
   providing 150 a second virtual reality content to the user configured to reduce the concentration level of the at least one biomarker in the sweat of the user, when providing the first virtual reality content to the user is interrupted.
29. The computer-implemented method 100 according to embodiment 28, wherein the second virtual reality content comprises a virtual reality generic artmaking task, a virtual reality drawing and/or painting tutorial, a virtual reality free drawing session, a virtual reality pottery session, and/or a virtual sculpting session.
30. The computer-implemented method 100 according to embodiment 28 or 29, further comprising
   adjusting 160 the second virtual reality content, when the concentration level is not reduced at a predetermined rate.
31. The computer-implemented method 100 according to any one of the embodiments 28 to 30, wherein the second virtual content is based on the age of the user and/or skills of the user.
32. The computer-implemented method 100 according to embodiment 30, wherein adjusting 160 comprises simplifying a task to be solved by the user, improving the comprehensibility of the second virtual reality content, and/or temporal extending of the virtual reality content.
33. The computer-implemented method 100 according to any one of the embodiments 26 to 32, further comprising calibrating 200 before providing 110 the first virtual reality content to the user, the calibrating 200 comprising
   providing 210 a plurality of virtual situations to the user;
   measuring 220 the concentration level of at least one biomarker in the sweat of the user corresponding to each virtual situation of the plurality of virtual situations by the sensor; receiving 230, via an input interface, from a user an estimation of the stress level felt by the user corresponding to each virtual situation of the plurality of virtual situations; matching 240 the estimation of a stress level felt by the user with the concentration level measured by the sensor corresponding to each virtual situation of the plurality of virtual situations;
   determining 250 a third threshold concentration level corresponding to a stress level above which the user has experienced a stress level in a smaller number of virtual situations that is greater than a stress level experienced by the user in a greater number of other virtual situations of the plurality of virtual situations and/or
   determining 260 a fourth threshold concentration level corresponding to a stress level below which the user has experienced a stress level in a smaller number of virtual situations that is smaller than a stress level experienced by the user in a greater number of other virtual situations of the plurality of virtual situations;
   storing 270 the matching, the third threshold concentration level and/or the fourth threshold concentration level in a storage medium;
34. The computer-implemented method 100 according to embodiment 33, wherein the calibrating 200 is enabled in predefined time intervals or based on a quality criterion derivable from the results of measuring the concentration level of the at least one biomarker.
35. The computer-implemented method 100 according to embodiment 33 or 34, wherein the first threshold concentration level equals the third threshold concentration level, is below the third threshold concentration level, or is above the third threshold concentration level.
36. The computer-implemented method 100 according to any one of the embodiments 33 to 35, wherein the range between a third threshold concentration level and the fourth threshold concentration level forms a range being suitable for a user for learning purposes.
37. The computer-implemented method 100 according to any one of the embodiments 33 to 36, wherein the second threshold concentration level equals the fourth threshold concentration level, is below the fourth threshold concentration level, or is above the fourth threshold concentration level.
38. The computer-implemented method 100 according to any one of the embodiments 32 to 37, wherein the first threshold concentration level and the second threshold concentration level are approximately the same.
39. The computer-implemented method 100 according to any one of the preceding embodiments, wherein the first virtual reality content comprises educational content.

## Claims

1. A virtual reality headset system (10), comprising
a display device (11);
a headband (12) coupled to the display device (11); and
a sensor (13) configured to measure an indication of at least one biomarker in the sweat of a user associated with a stress level of the user, when the user is wearing the virtual reality headset system (10).

2. The virtual reality headset system according to claim 1, wherein the at least one biomarker is a stress hormone.

3. The virtual reality headset system (10) according to claim 1 or 2, wherein the sensor (13) is configured to measure a concentration level of the at least one biomarker and to output a signal corresponding to the concentration level.

4. The virtual reality headset system (10) according to any one of claims 1 to 3, wherein the sensor (13) comprises a graphene layer with cortisol antibodies configured to bound cortisol in the sweat of the user allowing to output a signal corresponding to the cortisol concentration level.

5. The virtual reality headset system (10) according to any one of the preceding claims, wherein the sensor (13) is attached to the headband (12) or the display device (11) at a location where the sensor (13) contacts the skin of the user such as forehead, neck, back of the head, temples, chin area, nose, area around the eyes, and/or the upper head area.

6. The virtual reality headset system (10) according to any one of the preceding claims, wherein the display device (11) comprises one of a liquid crystal display, organic light-emitting diode display, or a light-emitting diode display.

7. The virtual reality headset system (10) according to any one of the preceding claims, further comprising a computer system (14) configured to operate the virtual reality headset system (10) and to receive a signal corresponding to the indication of the at least one biomarker.

8. The virtual reality headset system (10) according to claim 7, wherein the computer system (14) comprises a virtual reality input interface (15) configured to track movements of the user and to output a signal corresponding to the movements.

9. The virtual reality headset system (10) according to claims 7 or 8, wherein the computer system (14) comprises a communication interface (16) configured to allow the computer system to communicate with a computer and/or a mobile device.

10. A computer-implemented method (100) for monitoring at least one biomarker in the sweat of a user during using a virtual reality headset system (10), comprising providing (110) a first virtual reality content to the user through a virtual reality headset system (10);
measuring (120) the concentration level of the at least one biomarker in the sweat of the user through a sensor (13) coupled to the virtual reality headset system (10); interrupting (130) the providing (110) of the first virtual reality content to the user, when the concentration level of the at least one biomarker exceeds a predetermined first threshold concentration level; and
returning (140) to providing (110) the first virtual reality content to the user, when the concentration level reaches a level below a predetermined second threshold concentration level.

11. The computer-implemented method (100) according to claim 10, further comprising providing (150) a second virtual reality content to the user configured to reduce the concentration level of the at least one biomarker in the sweat of the user, when providing (110) the first virtual reality content to the user is interrupted.

12. The computer-implemented method (100) according to claim 11, further comprising adjusting (160) the second virtual reality content, when the concentration level is not reduced at a predetermined rate.

13. The computer-implemented method (100) according to claims 10, 11, or 12, further comprising calibrating (200) before providing (110) the first virtual reality content to the user, the calibrating (200) comprising
providing (210) a plurality of virtual situations to the user;
measuring (220) the concentration level of at least one biomarker in the sweat of the user corresponding to each virtual situation of the plurality of virtual situations by the sensor;
receiving (230), via an input interface (15), from a user an estimation of the stress level felt by the user corresponding to each virtual situation of the plurality of virtual situations;
matching (240) the estimation of a stress level felt by the user with the concentration level measured by the sensor corresponding to each virtual situation of the plurality of virtual situations;
determining (250) a third threshold concentration level corresponding to a stress level above which the user has experienced a stress level in a smaller number of virtual situations that is greater than a stress level experienced by the user in a greater number of other virtual situations of the plurality of virtual situations and/or
determining (260) a fourth threshold concentration level corresponding to a stress level below which the user has experienced a stress level in a smaller number of virtual situations that is smaller than a stress level experienced by the user in a greater number of other virtual situations of the plurality of virtual situations;
storing (270) the matching, the third threshold concentration level and/or the fourth threshold concentration level in a storage medium;

14. The computer-implemented method (100) according to claim 13, wherein the first threshold concentration level equals the third threshold concentration level, is below the third threshold concentration level, or is above the third threshold concentration level.

15. The computer-implemented method (100) according to claim 13 or 14, wherein the second threshold concentration level equals the fourth threshold concentration level, is below the fourth threshold concentration level, or is above the fourth threshold concentration level.
